# EUROPEAN PATENT SPECIFICATION

(11) **EP 2 834 750 B1**
(45) Date of publication and mention of the grant of the patent: **13.12.2017**
(21) Application number: 12715882.2
(22) Date of filing: 02.04.2012
(51) Int. Cl.: G06F 17/00, A61B 5/107

(54) **METHOD FOR ESTIMATING THE SHAPE OF AN INDIVIDUAL EAR**
VERFAHREN ZUM SCHÄTZEN DER FORM EINES EINZELNEN OHRS
PROCÉDÉ POUR ESTIMER LA FORME D'UNE OREILLE INDIVIDUELLE

(43) Date of publication of application: 11.02.2015
(73) Proprietor: Sonova AG, 8712 Stäfa (CH)
(72) Inventor: ROTH, Samuel, Hans, Martin, CH-8634 Hombrechtikon (CH); LEUTHOLD, Markus, CH-8712 Stäfa (CH)
(74) Representative: Troesch Scheidegger Werner AG
(86) International application number: PCT/EP2012/055972
(87) International publication number: WO 2013/149645

(56) References cited:
- EP-A2- 1 345 470
- US-A1- 2004 107 080
- US-A1- 2008 143 712
- US-A1- 2010 191 541
- US-B1- 7 050 876

## Description

### BACKGROUND

The present invention relates to a method for estimating the shape of an individual ear, and a method for manufacturing a custom fitted hearing device according to this estimation. Furthermore, the invention relates to methods for optimising In The Ear (ITE) shell design, Behind The Ear (BTE) or Canal Receiver Technology (CRT) housing design, and BTE tube design to persons within an identifiable population group

By "hearing device", a device is understood, which is worn in or adjacent to an individual's ear with the object to improve the individual's acoustical perception, or as a communication device. Such improvement may also be barring acoustic signals from being perceived in the sense of hearing protection for the individual. If the hearing device is tailored so as to improve the perception of a hearing impaired individual towards hearing perception of a "standard" individual, then we speak of a hearing-aid device. A hearing device may also be swim plugs, or in-ear headphones for a communication device or for music reproduction. With respect to the application area, a hearing device may be applied behind the ear (BTE) or in the ear (ITE), this latter encompassing the various styles of Full shell, Half shell, Canal, Mini Canal, CIC (Completely In the Canal), MIC (Micro in the Canal), and IIC (Invisible In the Canal).

Such devices are typically custom fitted to an individual. The traditional method for this involves taking an impression of the relevant part(s) of the individual's ear (such as the ear canal and/or concha), making a mould from the impression, casting the shell of the hearing device using the mould, placing of the components and faceplate in the shell, and final finishing of the shell. This procedure is extremely labour-intensive and relies on a high degree of skill on the part of the technician carrying it out.

The manual process was brought to the digital domain, taking scanned impressions as the starting point for digital modelling of the shell, which then is directly 3D-printed by means of a Rapid Prototyping or CNC device. While more controlled and more reproducible, much of the digital process remained manual, still needing skilled operators.

US 2004/0196995 describes a partially automated process whereby an impression is scanned, digitally processed so as to determine a characteristic feature, and then the model so produced is sent to a CNC machining station for fabrication of the shell in dependency of the characteristic feature.

US 2008/0143712 proposes a speculative knowledge-based method incorporating selecting a nearest-fit predetermined stored shell model to a scanned impression of part of an individual's ear. This nearest-fit model is selected from a series of predefined stored shell models and then is amended to match the scanned impression. However, the stored shell models may or may not be particularly similar to the desired end shape and thus may not give desirable results. To compensate this, the method also foresees that, in the case of no good match, generalised binaural modelling is performed, which rather negates the advantages of the main focus of the method. In any case, this document is highly speculative as to how the knowledge-based method is carried out, and does not provide any concrete realisation paths, particularly where automated extraction and classification of extracted features is concerned.

US 7,050,876 B1 discloses a method including operations to generate a first digital representation of a positive or negative image of at least a portion of an ear canal of a subject. A second digital representation of a hearing-aid shell is then generated having a shape which conforms to the ear canal of the subject. This second digital representation may be derived directly or indirectly from at least a portion of the first digital representation. Operations are then performed to print a hearing-aid shell that conforms to the ear canal of the subject, based on the second digital representation. Templates may be used to facilitate generation of the second digital representation. In particular, the operation to generate the second digital representation may comprise modifying a shape of the first digital representation to more closely conform to a shape of a digital template of a hearing-aid shell and/or modifying the shape of the digital template to more closely conform to the shape of the first digital representation. This digital template may be one of a plurality of possible templates retained in a library that is accessible and scannable by the CAD workstation in order to obtain a template having a highest degree of initial match to the first digital representation.

The object of the present invention is thus to improve upon the solutions in the prior art, and thereby provide concrete realisation paths to a universal, statistically-based methodology for estimating the shape of an individual ear, e.g. for the purposes of manufacturing a customised hearing device, in as automated a fashion as possible, requiring as little manual interaction as possible. The output of this method can then be used as input data for fabrication of custom shells, earpieces and similar.

Further objects of the invention are to apply the same knowledge to optimise ITE shell design, BTE/CRT housing design, and BTE tube design to fit a majority of individuals.

### SUMMARY OF THE INVENTION

The invention is defined by the independent claim, whereby the dependent claims define preferred embodiments. In particular, the above-mentioned advantages are achieved by a method of estimating the shape of an individual ear comprising the steps of:
a) determining at least part of the shape of the individual ear, e.g. by taking an impression, or similar, the shape being determined over a first extent and constituting a determined shape;
b) taking a predefined template shape determined by statistical analysis of a plurality of previously measured ear shapes according to a statistical model, the template shape having a second extent, which is greater than the first extent;
c) generating an estimated shape corresponding to an estimated representation of the shape of the individual ear over combination of the first extent and the second extent (that is to say the largest extent possible by combining both extents, in other words the "union", or Boolean "or" of the two shapes) by modifying the template shape to substantially match the determined shape over the intersection of the first extent and the second extent (i.e. the overlap of both extents), within a predefined tolerance, for instance by means of a linear or non-linear optimization of template parameters resulting in rigid and/or non-rigid alignment of the template, e.g. via one or more approaches selected from warping, morphing, projecting, or any sort of rigid or non-rigid registration.

This matches the template onto the determined shape to estimate the shape of the individual ear which has an extent greater than the first extent. This permits infilling of holes, gaps, missing parts etc. on the determined shape, and extrapolation of the determined shape into areas not reached by the first extent. By using a predefined template shape determined by statistical analysis, the chances of the modelling process giving the desired result is significantly increased since this template ear will by definition have a lot in common with any given ear, and eliminates the step of identifying and selecting a "closest fit" from a potentially rather large database of shell models. This thereby speeds up the process compared to that of US 2008/0143712. This method is naturally carried out by means of a computer program on a programmable computer.

In an embodiment, the template shape is matched to the determined shape by mutually aligning the determined shape and the predefined template shape and deforming the predefined template shape to substantially match the determined shape over the intersection of the first extent and the second extent within the predefined tolerance. "Mutually aligning" the determined shape and the predefined template shape can be either carried out by aligning the determined shape with the predefined template shape, or by aligning the predefined template shape with the determined shape. This provides a concrete and efficient "matching" method.

In an embodiment, the estimated shape is re-projected or wrapped onto the measured shape such that data from the estimated shape is used to interpolate and/or extrapolate to provide fill-in data for inhomogeneities caused by holes, fragmentary data, outlying points, or missing parts of the measured shape. This can then optionally be combined with the measured shape to create an infilled shape of the measured ear, which permits essentially taking the measured shape where it is of "good" quality, thereby incorporating its fine detail where possible, and then extrapolating and interpolating from the estimated shape to fill-in missing data, missing parts, or incomplete parts. Alternatively, detail from the determined shape not captured in the estimated shape is incorporated into the infilled shape which achieves the same results.

According to the invention, the determination of the previously measured ear shapes used to create the template is carried out by at least one of laser scanning of impressions, (allowing usage of current methods and technology), MRI or CT scanning (which allows application of volumetric, high quality, complete data covering the whole ear including the pinna and the space behind it, this latter being particularly useful for a BTE application), or direct ear scanning (which gives exceptionally precise, high quality data). It is important for the data relating to the previously measured ear shapes to be as complete as possible, that is to say to cover as much of the ear as necessary at as high a resolution as practical, and the data to be as high quality as possible.

According to the invention, the extrapolation is used to estimate the shape of the ear canal up to the eardrum or to within less than 5 mm from the eardrum, which is then used for estimating the residual volume, the Real Ear to Coupler Difference (defined as the difference in decibels, as a function of frequency, between the sound pressure level at the eardrum and the sound pressure level in a 2cc coupler, for a specified input signal), the Open Ear Gain (amplification due to the outer ear, without insertion of an earmold or hearing aid), and other properties useful in the design of hearing devices.

In an embodiment, the first extent is less than the second extent by virtue of the first extent covering less of the shape of an ear than the second extent, which is the case when e.g. volumetric CT or MRI data of a whole ear is used to extrapolate or fill-in a comparatively smaller determined shape emanating from an impression or a direct scan of part of an ear canal. Alternatively, the first extent is less than the second extent by virtue of missing parts, holes, fragmentary data, outlying points, or missing parts, which can then be corrected and filled in by interpolation and extrapolation of the template.

In an example not falling under the scope of the claims, the second extent is less of the first extent by virtue of the second extent covering less of the shape of an ear than the first extent. This is useful in the case when a "shorter" template is needed to fill-in and interpolate for a longer determined shape emanating from an impression or from a direct scan.

In an embodiment, the determined shape is sampled at a lower resolution than the template shape. This speeds up processing since e.g. scanning of an impression or direct ear scanning for the determined shape can be effected quicker, and the remaining resolution can then be interpolated from a high-quality, high-resolution template, e.g. emanating from an MRI or a CT scan.

In an embodiment, the template shape is provided with meta data corresponding to features, such as identifiable geometric features, i.e. shape features from the set of ear canal bends, aperture of ear canal, concha, cymba, helix, crus, inter-tragal notch, anterior notch, tragus, anti-tragus, ear drum, strong curvature lines, and retention areas. Alternatively, this meta data could correspond to texture, softness of tissues, contour lines, underlying bony structures such as the transition between cartilaginous and bony ear canal parts, or the jawbone influence area. All of these features are useful in the design of hearing aids, and by incorporating them into the template shape means that, when the estimated shape is created, the meta data can be transferred onto the estimated shape or the infilled shape, and thus these features can be be automatically marked and identified on the estimated and/or infilled shape as appropriate. Additional or alternative meta data can correspond to intended positions of hearing aid components such as faceplates, receivers, microphone or microphones, hybrids, batteries, telephone coils, inductive coils, antenna coils, reed switches, capacitors, vents, and wax guards. Once the estimated shape is created, this meta data will be automatically transferred onto it, deforming with the template, since it is "attached" thereto, resulting in predefined component positions and thereby automated component placement without requiring complicated and time-consuming algorithms. The primary advantage of this is that, given that the features are pre-identified on the template by the meta data, when the template is warped onto the determined shape, the meta data deforms with the template, and thus the features are automatically identified on the determined shape. This thus achieves automated feature recognition on the determined shape.

Alternatively, or in addition, the meta data can correspond to a pre-modelled deformable shape of at least part of the hearing device. This could, for instance, correspond to the distal and proximal surfaces of the shell of a hearing device, such as e.g. earmould style templates or ITE shell templates, or contours defining such earmold or shell shapes, which could result in automated shell design when this meta data is transferred onto the estimated shape. The estimated shape provided with this meta data could then serve as a statistically-based high-quality starting point for further manual improvement or even very simply be sent to a 3D printer or CNC machining station which could then produce the shell or earmold without further, or with minimal, technician intervention.

In an embodiment, the template shape is created based on averaging a plurality of previously measured shapes according to the statistical model. This results in far greater accuracy than the above-discussed prior art implementation, since a statistically determined average ear will necessarily be more representative of ears in general and will be free of any highly unusual characteristics or features.

In an embodiment, this template shape is selected from a plurality of template shapes each previously generated by the same method described above and corresponding to templates for a priori identifiable population groups such as male, female, age ranges, ethnicity, or any combination thereof. This further improves the accuracy of the estimated shape for very little technician input since it enables an average based on a particular identifiable phenotype to be used rather than an entirely generic average shape, the technician selecting the requisite template from the plurality of previously-generated templates based on a priori knowledge of the person from whom the impression was taken. A further advantage of this is that, for each group or "class", the "average" shape will be less diluted by features from other classes as would be the case with a single globally-averaged template, so the "class" template will be more representative of that particular "class".

In an embodiment, the template shape corresponds to at least part of the ear canal and/or the outer ear including the portion situated to the rear of the pinna. When the template shape corresponds to at least part of the ear canal and/or the outer ear, it is particularly suited to providing data for hearing devices penetrating into the ear canal. When the template corresponds to the rear of the pinna, it is particularly suited for defining the shape of BTE housings, or sound tubes or wire tubes.

In an embodiment, the statistical model is built by multidimensional Principal Component Analysis (PCA), also known as Covariance Analysis, Karhunen-Loeve Transform, or Hotelling Transform. This well-understood mathematical approach is particularly suited for the proposed method, since it is accurate and efficient. Other methods are of course not excluded from the scope of the invention.

In an embodiment, the statistical model is a size-and-shape model incorporating size as a trait of shape, or a shape-only model in which the plurality of previously measured ear shapes are normalised in size. This provides several possible options for the statistical model according to the needs of the technician.

In an embodiment, the determined shape is incorporated into the generation of the template shape for the next time the method is carried out, thereby iteratively improving the template shape by generating it from an ever-increasing population of measured shapes.

In an embodiment, the predetermined average template shape is a Template Active Shape Model determined by:
- taking a plurality of digitised measured sample ear shapes;
- defining a plurality of landmark points on each of said digitised measured sample ear shapes, each landmark point corresponding to an identifiable feature common to all sample ear shapes;
- aligning the measured sample ear shapes based on the landmark points;
- creating a mean shape by taking the mean of the landmark points.
- from the mean shape, defining a template mesh with a regular mesh structure and a resolution adequate for both capturing shape-relevant features and allowing for Principal Component Analysis;
- in turn, deforming the template mesh to substantially match each measured sample ear shape within a predefined tolerance, thereby defining a set of Principal Component Analysis meshes, the representations of which are in one-to-one correspondence, i.e. have an identical mesh structure;
- optionally, regularising the said Principal Component Analysis meshes;
- aligning said Principal Component Analysis meshes and then computing a mean PCA mesh of all Principal Component Analysis meshes of the said set;
- running a Principal Component Analysis on the Principal Component Analysis meshes around the mean PCA mesh to generate a matrix of eigenvectors;
- reducing dimensionality in the matrix of eigenvectors by identifying relevant PCA modes in the matrix of eigenvectors and eliminating corresponding columns of the matrix so as to obtain a more compact representation still capturing sufficient variation in the data, resulting in the PCA system matrix;
- constituting the Template Active Shape Model by the PCA system matrix: and the mean PCA mesh.

This is a particularly efficient and reliable method of constituting the template shape, and results in a format for describing the template shape which is data-compact. Alternative embodiments are possible for at least partially defining the template mesh, such as establishing dense correspondence by e.g. structure-preserving, minimum distortion, or isometric mappings. Any embedding of surfaces on one another by optimising a metric (e.g. least distortion or isometry) is also envisaged for at least partially defining the template mesh. Similarly, PCA is one (linear) way of analysing the data. Alternative embodiments include non-linear and multi-linear alternatives and generalizations of the concept (principal curves and manifolds, principal geodesic analysis, MPCA etc). In an embodiment, the sample ear shapes and/or Principal Component Analysis meshes and are aligned using Generalised Procrustes Analysis (GPA), with or without optimization for scale. This allows efficient alignment of the shapes or PCA meshes.

In an embodiment, the template mesh is defined by the following steps:
- taking the measured sample ear shape with landmarks closest to the mean shape;
- warping, for instance by Thin-plate Spline warping, the above-mentioned sample ear shape to the mean shape;
- in the case of incomplete data, cutting the sample ear shape open at the portion corresponding to the ear canal and optionally also corresponding to the helix;
- further pruning the sample ear shape to the extent of the least common denominator (i.e. the part of the surface or shape that is covered by all samples in the training set, in other words the logical intersection of all surfaces of the samples in the training set) of the plurality of digitised measured sample ear shapes;
- decimating and re-meshing the mean shape to a resolution adequate for both capturing shape-relevant features and allowing for Principal Component Analysis.

This allows efficient and accurate definition of the template mesh in a format is suitable for PCA analysis. The skilled person fully understands for a given case how fine the mesh may be in order to capture needed detail while still being course enough to permit efficient PCA analysis, since this approach is well-known and well-understood. Furthermore, as above, PCA is one (linear) way of analysing the data. Alternative embodiments include non-linear and multi-linear alternatives and generalizations of the concept (principal curves and manifolds, principal geodesic analysis, MPCA etc).

In an embodiment, the deformation of the template mesh is carried out at least partially by Thin-plate Spline warping.

This allows very accurate deformation of the template mesh with an efficient method.

In an embodiment, every time the method is carried out, the new determined shape is incorporated into the determination of the Template Active Shape Model. This causes, over time, the Template Active Shape Model to conform more closely to a theoretical "average ear" representative of any given population, improving the overall method over time. As above, it may be desirable to define the average ear a priori according to sex, ethnicity, or any other identifiable characteristic and thereby create a different "average" for each identifiable grouping.

In an embodiment, the step of aligning the determined shape with the average template shape is carried out by a rigid Iterative Corresponding Points approach, and the step of deforming the average template shape so as to substantially match the determined shape may be carried out by a non-rigid Iterative Corresponding Points registration approach. This again provides efficient processing. As "correspondence", we understand either "closest" in terms of Cartesian proximity, or in terms of the closest intersection found along surface normal direction, often referred to as "normal shooting".

In an embodiment, the non-rigid Iterative Corresponding Points registration comprises the following steps, starting with the template as the current registration:
- updating the correspondence between the determined shape and the current registration;
- estimating parameter changes induced by the updated correspondence;
- updating the optimisation parameters according to the estimated parameter changes;
- computing a new current registration based on the current optimisation parameters;
- repeating the steps as necessary until the mean square error difference between the new current registration and the determined shape drops below a predefined tolerance threshold, thereby resulting in the matched shape.

Alternatively, the non-rigid Iterative Corresponding Points registration comprises the following steps, starting out from the undeformed template shape in neutral pose as the current registration:
a) rigidly aligning the current registration to the determined shape via Iterative Corresponding Points, thus updating the pose;
b) updating the Corresponding Points correspondence between the current registration and the determined shape as defined by the Iterative Corresponding Points registration;
c) determining whether the mean square error between the current registration and the Corresponding Points computed in the previous step is below a predefined threshold, and if yes outputting the current registration as the estimated shape and stopping the non-rigid Iterative Corresponding Points registration;
d) transforming the Corresponding Points back to neutral pose;
e) deforming the Template Active Shape Model to match the Corresponding Points in neutral pose;
f) updating the current registration with the deformed template and returning to step a).

These two variants permit efficient matching of the template shape to the actual shape of the measured ear impression.

In an embodiment, outlying points are eliminated from the registration. This improves the quality of the final shape and therefore will improve the fit of a custom hearing device whose shape is calculated according to the method.

In an embodiment, manual intervention is effected upon the definition of the landmark points. This allows a relatively minor amount of manual intervention to improve the identification of the landmark points compared to what an automated algorithm can achieve, thereby improving the accuracy of the method.

The invention further concerns a computer program adapted to carry out the steps of the above-mentioned method(s), and an electronic storage medium comprising the said computer program stored thereupon.

The invention further concerns a method of manufacturing a custom fitted hearing device comprising the steps of:
- determining the estimated shape and/or the determined shape according to any of the above-mentioned methods;
- manufacturing a shell of the custom fitted hearing device such that at least the portions of the shell destined to be in contact with part of the ear of the wearer is conformed according to the estimated shape and/or the infilled shape as appropriate.

This enables application of the above method to physical manufacture of custom hearing devices, enabling them to be manufactured faster, more cheaply, and more simply than present methods.

In an embodiment, the shell manufactured by the method is a shell of an ITE, CRT (also known as RIC or RITE), BTE or HPD device, a swim plug, or a custom earphone, enabling adaptation of the method to all of the various types of hearing device.

A further aspect of the invention is a method for optimising a BTE or CRT housing, or a BTE tube to the shape and space available behind the ear of persons within an identifiable population group, which may be the group comprising all persons, comprising the steps of:
a) taking a predefined template shape determined by statistical analysis of a plurality of previously measured ear shapes according to a statistical model, the template shape corresponding to at least the shape and space behind the ear;
b) defining a defined shape of at least part of the shape of a BTE or CRT housing or a BTE tube corresponding to the predefined template shape. This enables the previously described knowledge-based method to be applied to defining a "best fit" BTE or CRT housing or BTE tube that will fit a majority of people within a given identifiable population group. This method is naturally carried out by means of a computer program on a programmable computer. In an embodiment of this method, the predefined template shape is defined exactly the same as above, although of course the measured ear shape data incorporates shape information from behind the pinna.

In an embodiment, the identifiable population group is at least one of male, female, age ranges, or ethnicity, which enables a good "average" fit to be achieved without significant manual intervention in the process.

The invention further relates to manufacturing a BTE or CRT housing or a BTE tube according to the determined shape determined above, enabling the creation of housings likely to fit well for most members of the given population group.

The invention further relates to a method of optimising an ITE shell to the shape of the ear of persons within an identifiable population group, which may comprise the group of all persons, comprising the steps of:
a) taking a predefined template shape determined by statistical analysis of a plurality of previously measured ear shapes according to a statistical model, the template shape corresponding to at least the part of the ear in which the ITE shell is destined to be situated in use;
b) defining a defined shape of at least part of the shape of an ITE shell corresponding to the predefined template shape.

This enables the previously described knowledge-based method to be applied to defining a "best fit" ITE housing that will fit a majority of people within a given identifiable population group. This method is naturally carried out by means of a computer program on a programmable computer. In an embodiment of this method, the predefined template shape is defined exactly the same as described above.

As above, in an embodiment, the identifiable population group is at least one of all persons, male, female, age ranges, or ethnicity, which enables a good "average" fit to be achieved without significant manual intervention in the process.

Finally, the invention relates to a method of manufacturing an ITE shell comprising the step of fabricating the ITE shell according to the defined shape determined above, enabling the creation of ITE shells likely to fit well for most members of the given population group.

### DESCRIPTION OF THE DRAWINGS

The invention is illustrated by means of non-limiting specific embodiments illustrated in the figures and explained in more detail below:
Figure 1 shows a flow diagram for generating the template shape;
Figure 2 shows a high quality Template Mesh;
Figure 3 shows the influence of the first three PCA modes on template shape;
Figure 4 shows a first variant of non-rigid Template Active Shape Model (TASM) matching;
Figure 4a shows examples of outlying points;
Figure 5 shows a second variant of nonrigid TASM matching;
Figure 6 shows a flow diagram for incorporating a new impression into the template model;
Figure 7 shows an estimated shape matched to a measured shape; and
Figure 8 shows a TASM with annotated feature lines.

### DETAILED DESCRIPTION OF THE DRAWINGS

Although the below mentioned embodiments use Principal Components Analysis, other mathematical methods are envisaged or and not be construed as excluded from the scope of the invention except where doing so would cause contradiction. Naturally, the method(s) will be carried out on a computer as a computer-based method.

The mathematics underlying the detailed embodiments of the invention is expounded in the following publications:
R. R. Paulsen, Statistical Shape Analysis of the Human Ear Canal with Application to In-the-Ear Hearing Aid Design, Thesis, Informatics and Mathematical Modelling, Technical University of Denmark, Building 321, Richard Petersens Plads, DK-2800 Kgs. Lyngby, 2004.
D. C. Schneider, P. Eisert, Fast Nonrigid Mesh Registration with a Data-Driven Deformation Prior, NORDIA workshop, International Conference on Computer Vision ICCV, Kyoto, 2009.
D. C. Schneider, P. Eisert, Fitting a Morphable Model to Pose and Shape of a Point Cloud, Vision, Modeling and Visualization VMV, Braunschweig, 2009.

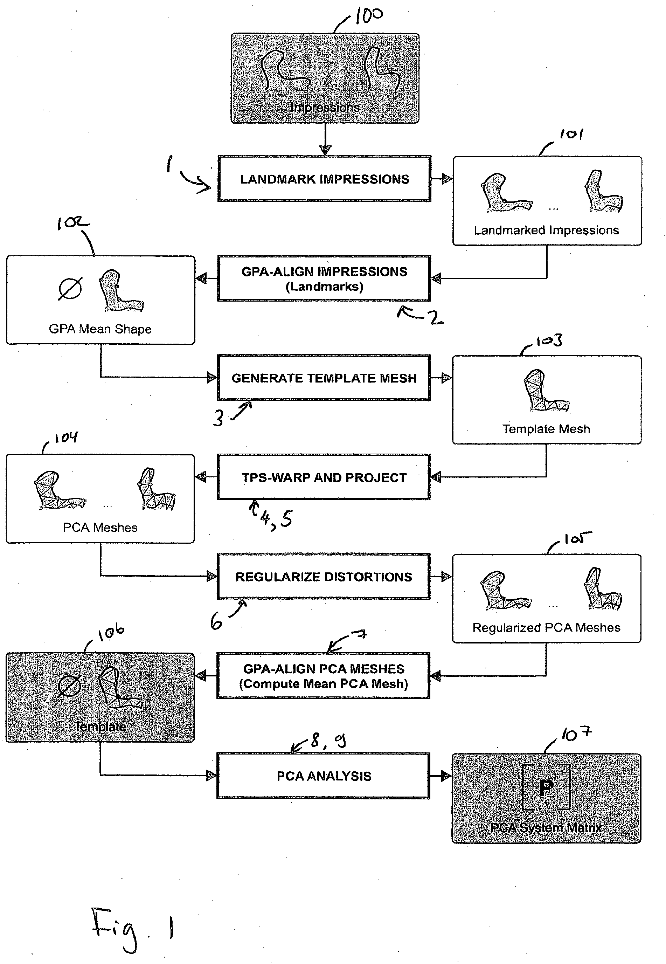

Figure 1 shows a flow diagram for creating the template shape.

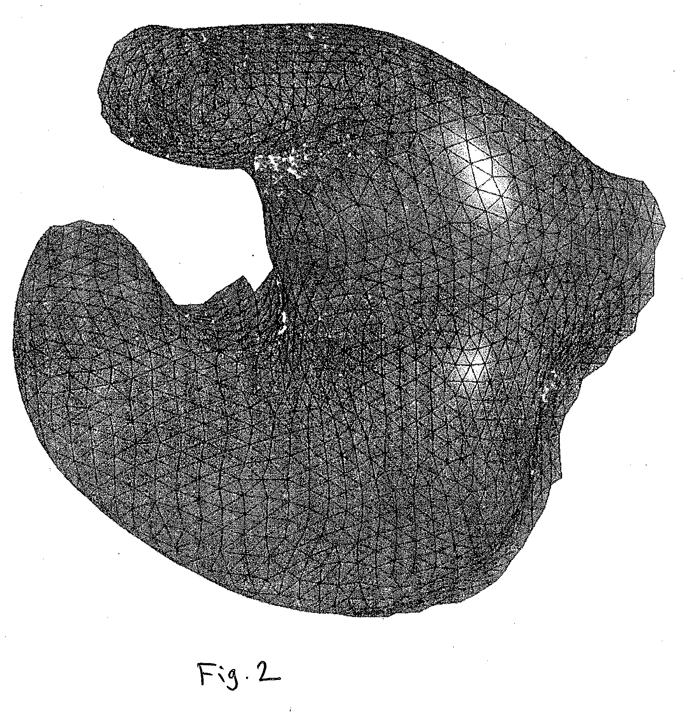

The generation of the template shape can be stated as a step-wise procedure:
In step 1, a training set of M "impressions" 100 is manually landmarked, i.e. measured ear shapes, with features (points or contours as a sequence of points). The landmarks are illustrated as small circular points on the shapes in box 101.
   a. It is important for the impressions 100 to be complete, i.e. to comprise a complete set of features (all landmarks).
   b. Landmarks must be of good correspondence, i.e. provide a "homomorphism" between impressions. To put it simply, a feature landmark annotated on each training impression must closely correspond to the same feature on all other impressions.
In step 2, all landmarked impressions are aligned in a common coordinate system via Generalized Procrustes Analysis (GPA). GPA is set up to only change rotation and translation, but not scale, since scale is considered part of the shape information of ears.
   a. A reference impression is chosen from among the training set.
   b. All other impressions of the training set are aligned to the current reference impression.
      Alignment is formulated as a least squares problem, attempting to find a minimum Procrustes distance (sum of squared distances between corresponding landmarks on the impressions). Possible solutions comprise Singular Value Decomposition (SVD)-based Procrustes approaches or quaternion-based approaches solving the equivalent problem of absolute orientation).
   c. The mean shape of the aligned training set is computed (mean of landmarks).
   d. If the Procrustes distance between the mean shape and the chosen reference is above a certain threshold, i.e. is within a predefined tolerance, the reference is set to the computed mean shape and continue to step b.
   e. The found mean shape is the GPA Mean Shape 102.
In step 3, from the GPA Mean Shape 102, a Template Mesh 103 with N vertices is created. It should have a regular mesh structure and a resolution dense enough to capture shape-relevant features while still coarse enough to allow for Principal Component Analysis (PCA). Figure 2 illustrates a template mesh exhibiting these required properties. The template mesh should again be complete, i.e. comprise all relevant features (landmarks), but should also be the "least common denominator" in that it only comprises shape present on all training impressions. A possible approach is the following:
   a. The training impression with landmarks closest to the GPA Mean Shape 2 is taken.
   b. It is then Thin-plate Spline (TPS) warped to the GPA Mean Shape 102.
   c. It is cut open at the canal (and possibly helix). Cutting open is only needed for incomplete impression data, such as scanned silicone impressions, but not for complete ear shape data, e.g. from segmented CT scans.
   d. It is then decimated, pruned, and re-meshed to meet the above mesh requirements.
In step 4, the Template Mesh is Thin-plate Spline (TPS) warped to each impression according to landmark correspondences (the Template Mesh having landmarks from the GPA Mean Shape 102).
In step 5, all vertices of the TPS-morphed Template Mesh are reprojected to each training impression. This results in what we call the PCA Meshes 104.
In step 6, if necessary, warping and projection distortions are regularized via Markov Random Field (MRF) regularization, refining the PCA Meshes, resulting in regularized PCA meshes 105.
In step 7, a Mean PCA Mesh of all PCA Meshes, also known as the Template 106, is computed via an additional GPA on the PCA meshes (thereby also computing the mean).
In step 8, a Principle Component Analysis (PCA) is run on the PCA Meshes. Using a centroid representation around the Mean PCA Mesh computed above, Singular Value Decomposition (SVD) results in an orthonormal matrix of eigenvectors of the covariance matrix with corresponding eigenvalues stored in the diagonal of the matrix. The PCA transform preserves dimensionality, i.e. has the same number of principal components as in the original data.
In step 9, as the last step, dimensionality is reduced by identifying relevant PCA Modes in the matrix of eigenvalues and omit corresponding columns of the matrix of eigenvectors in order to obtain a more compact representation still capturing relevant variation in the data. The such reduced matrix results in the PCA System Matrix 107.

The outlined sequence of steps results in a Template Active Shape Model (TASM) defined by the PCA System Matrix and the Mean PCA Mesh or Template. Multiplying the PCA system matrix with a vector of PCA Modes and adding the mean PCA mesh allows for computing a Deformed Template.

Setting the vector of PCA modes to zero yields the undeformed TASM, corresponding to the Template 106. In other words, we can generate shapes out of the template by setting weights, i.e. the PCA modes.

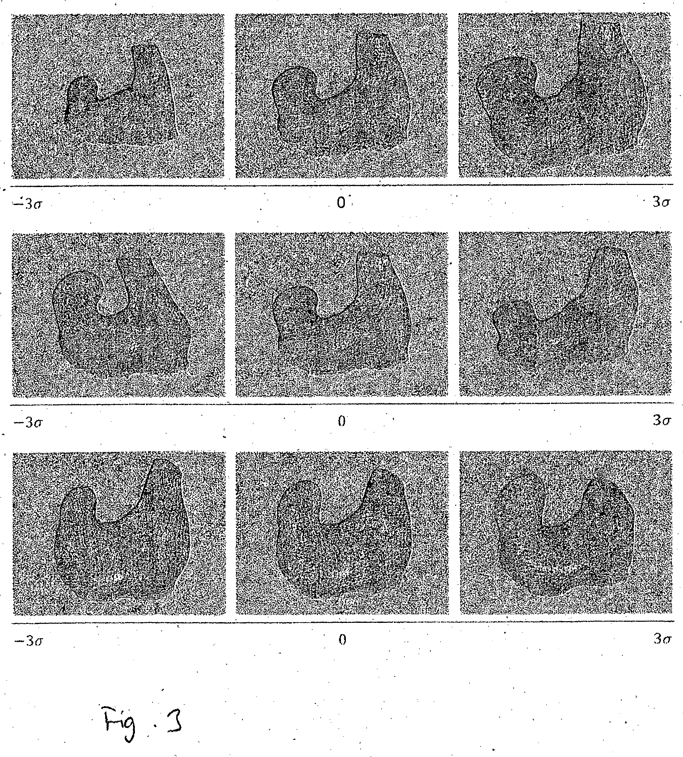

Figure 3 highlights the influence of the first three PCA Modes on the template shape. For each of the three modes, the figure shows the undeformed template in the middle, with a -3σ deformation weight on the left and 3σ on the right, respectively. The choice of 3σ (three standard deviations seen for that mode in the training set) ensures that the deformed template is kept within the space of allowed (and meaningful) shapes. The top row illustrates the effect of the first PCA mode, which mainly affects size, the middle row illustrates the effect of the second PCA mode, which mainly impacts the angle between the helix/cymba and the canal, and the bottom row illustrates the effect of the third PCA mode on canal length and twist.

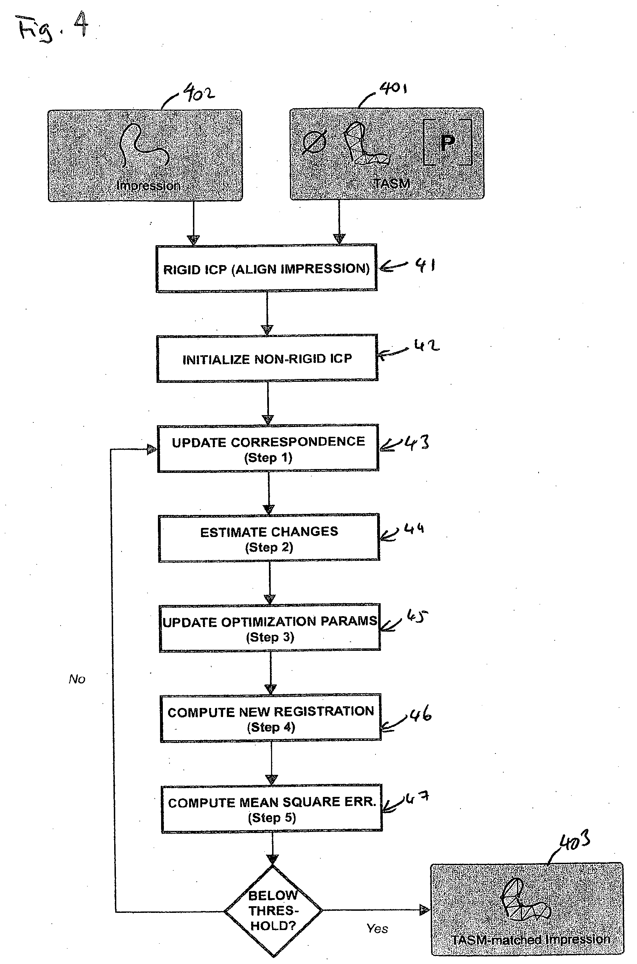

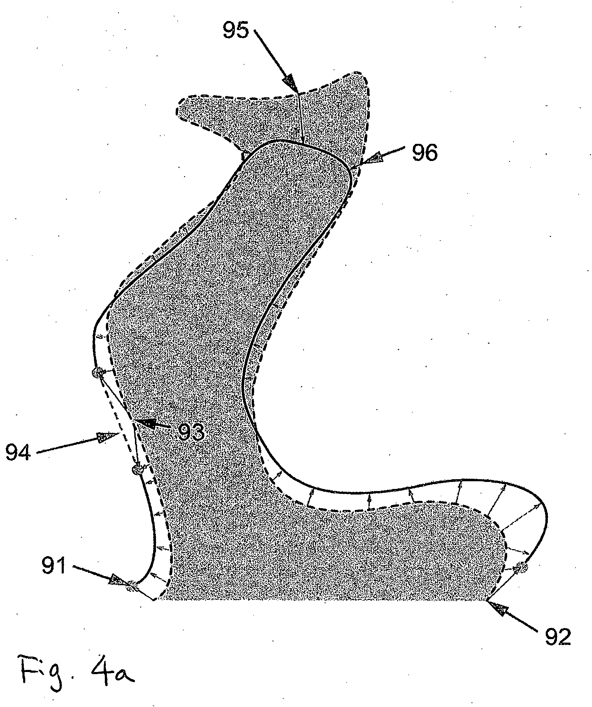

Figure 4 shows a first embodiment of matching the previously-generated TASM 401 to a newly measured determined shape 402, i.e. impression, taken from e.g. a prospective wearer of a hearing device.

Overall, matching the TASM and the impression can be regarded as an optimization problem, estimating a set of parameters such that they minimize a cost function. The set of parameters consists of the weights in the PCA Modes vector for a non-rigid registration part (deforming the TASM) and a rigid registration part for the pose (translation and rotation). The cost function is a surface distance between the deformed template shape and the new impression.

Firstly, in the rigid-registration step 41, the new impression (i.e. the determined shape) is aligned with the TASM by means of a rigid Iterative Corresponding Points (ICP) method, which aligns the impression with the TASM by translation and rotation. Rigid ICP is well-documented in the literature and need not be described further. By aligning the pose of the impression and the TASM, unnecessary warping of the TASM in the non-rigid registration step due to differences in pose is avoided, reducing errors and resulting in an estimated shape which is a more accurate representation of the real ear.

In step 42, non-rigid ICP registration is initialised based on starting with the undeformed TASM in a neutral pose with respect to the impression. The initial registration thus corresponds to the Template (mean PCA mesh).

In step 43, the correspondence (in the sense of standard ICP) is updated by identifying the corresponding points on the impression for each point of the current registration (which on the first run through of the method corresponds to the Template).

In step 44, the parameter changes induced by the updated correspondence are estimated.

In step 45, the optimisation parameters are updated based on the parameter changes found in step 44.

In order to keep the non-rigidly deformed template in the space of allowed (and meaningful) shapes, each mode's weight can be clamped, e.g. to be within three standard deviations 3σ seen for that mode in the training set.

In step 46, a new registration is computed based on the current optimisation parameters.

In step 47, the mean square error is calculated, and if it is above a predefined threshold, i.e. the match is not within a predefined tolerance, the method is repeated from step 43 until it is. Once the match is within the predefined tolerance, the TASM-matched impression 403 corresponding to the estimated shape of the individual ear over the desired extent is output.

If necessary, as part of the non-rigid registration, outliers can be eliminated, since non-rigid registration can suffer from incomplete data. In the present example, the new impression could have holes (scanning artifacts) or may cover a smaller ear shape area than the template (e.g. be shorter at the tip). It may also have failed to capture certain features of the ear entirely. Such missing data can have a negative effect on the registration result. Template points corresponding to missing surface parts would in this case get matched against distant points on the new impression, introducing large squared errors and thus resulting in unwanted strain in the optimization and possibly leading to distortions in the final match.

Three types of outliers can be distinguished:
1. Distance: Correspondences found between points above a certain distance threshold.
2. Border: Template points matched to surface borders on the impression.
3. Orientation: Correspondences found between template points and impression points that have strongly deviating surface normals, i.e. far off (anti-)parallel.

The 2nd class of outliers can either be due to holes (resulting in hole borders) or due to a smaller surface area on either the template or the new impression (actual surface border).

Figure 4a illustrates the three types of outliers, and shows the impression border 91, an outlier corresponding to the impression border 92, an outlier correspondence to a hole border 93 due to a hole 94, an outlier 95 above the distance threshold which has no corresponding shape, and an outlier 96 for bad normal correspondence.

In order to account for outliers, all needed classes of outliers are identified in a first step and equations corresponding to outlier points in the linear system are omitted in a second step.

Depending on the quality of the initial rigid registration, it can be problematic to prematurely exclude point correspondences identified as outliers, in particular for outlier types 1 and 3: the template can still deform during non-rigid ICP. This applies most if the initial correspondence is rather bad. A more sophisticated outlier elimination scheme would therefore wrap the above non-rigid ICP registration iteration into an outlier elimination loop, or even combine outlier elimination inside the ICP iteration with elimination in an outer outlier loop.

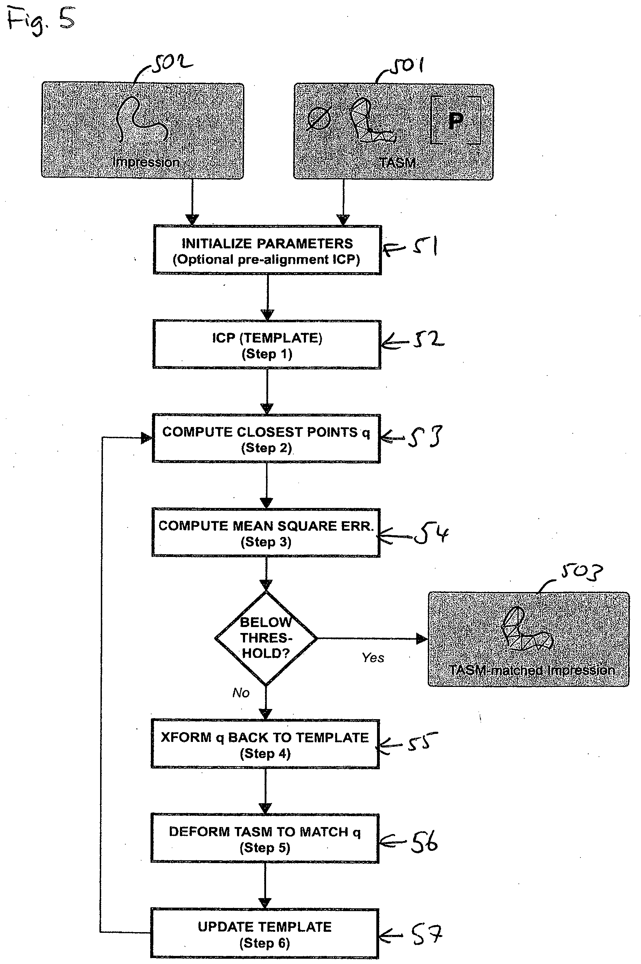

An alternative embodiment of non-rigid ICP registration is illustrated in figure 5. In this embodiment, the optimisation of the rigid-body transformation parameters and the non-rigid deformation of the template are interleaved into one combined optimisation. Another possibility which is foreseen but not illustrated is that the rigid pose and the non-rigid deformation could be separated into two subsequent steps and iterated to convergence.

In step 51, the parameters are initialised, with the undeformed TASM in neutral pose, with the template initialised to the mean PCA mesh. Optionally, pre-alignment ICP can be effected.

In step 52, ICP of the template is carried out by updating the transformation that minimises the distances between the impression and the initialised TASM, in the sense of standard ICP.

In step 53, corresponding points are identified on the impression for each point of the current registration, resulting in a shape q.

In step 54, mean square error is computed and compared with the threshold in the same manner as for the embodiment of figure 4. If the mean square error is below the threshold, the TASM-matched impression 503 is output, this impression corresponding to the estimated shape. If not, the method continues with step 55.

In step 55, the shape q is transformed back to the template, generating another shape q'.

In step 56, the TASM is deformed to match q' by back projecting q' into PCA space. As in the embodiment of figure 4, in order to keep the deformation in the space of meaningful shapes, each PCA mode's weight can again be clamped, e.g. within three 3σ (3 standard deviations).

In step 57, the template is updated according to the parameters calculated in step 56. From here, the method loops back to step 53 above.

It is noted that this method can either use a size-and-shape model, or it can also use a shape-only model including scale as a feature.

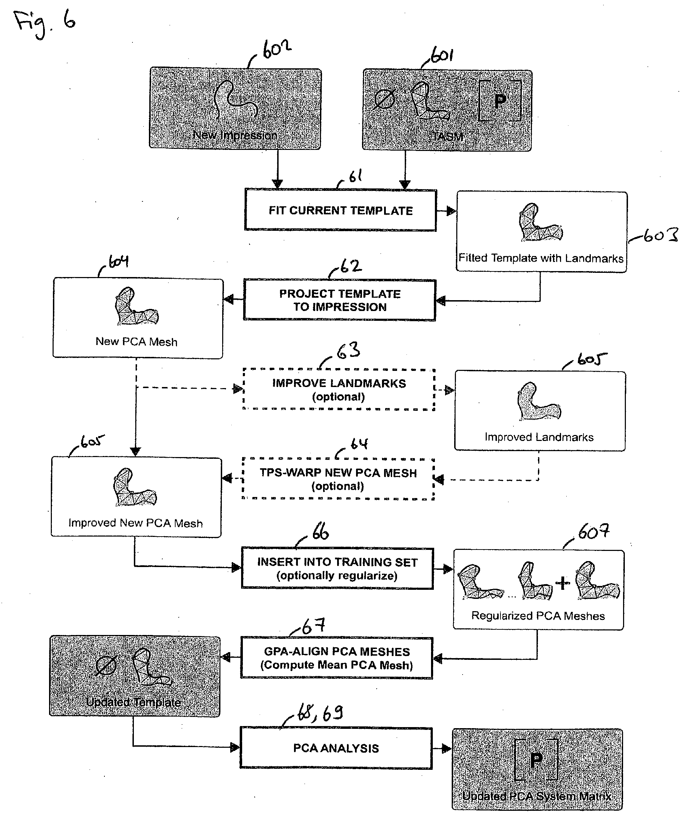

As was previously discussed with regards to figure 1, the initial generation of the template shape required manual placement of the landmark points. Once an initial Template Active Shape Model (TASM) has been created by the method illustrated in figure 1, it is possible to "grow" the TASM in a far more automated way, as illustrated in figure 6. The current template TASM 601 is fitted in step 61 to a new impression 602, as outlined above in reference to the methods of figures 4 and 5, resulting in a fitted template with landmarks 603. After fitting, the template is projected to the new shape in step 62. This defines a dense correspondence of the existing TASM with the new impression, and the result of this projection can be taken as a new PCA mesh 604, and the TASM can be updated via the insertion of the new PCA mesh 604 into the training set in step 66, during which it may optionally be regularised, resulting in regularised PCA meshes 607, and finally the TASM is updated by means of the process steps 7 to 9 as described in context with figure 1, referenced on figure 6 as steps 67-69 respectively.

Optionally, in step 63 the landmarks on the new PCA mesh 604 may be manually improved, resulting in improved landmarks 605, and this may then be optionally TPS warped in step 64 to create an improved new PCA mesh 605 which is then used in steps 66-69 in substitution for PCA mesh 604.

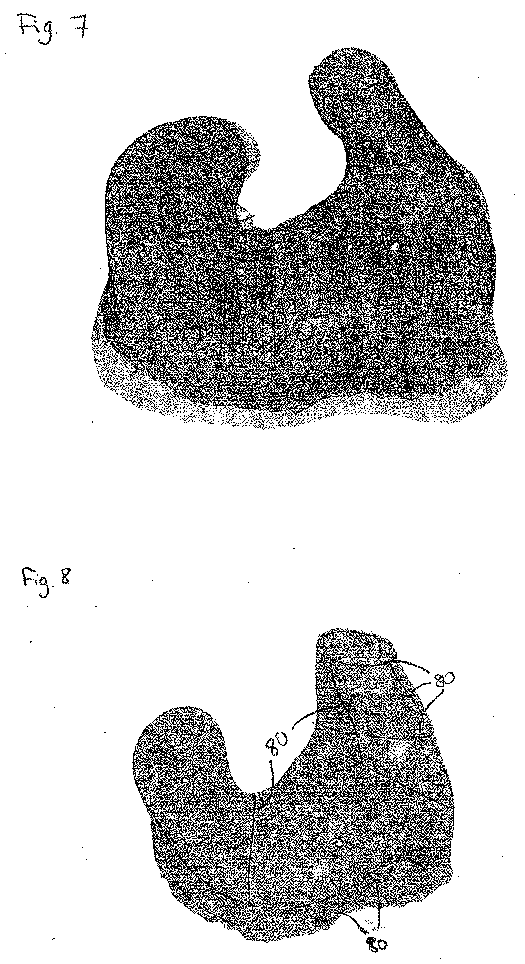

Figure 7 illustrates an example fitting of a TASM to a new impression. The overall shape is well matched, while certain fine details are not captured in the model. This can be resolved by backprojecting the fitted TASM back onto the original impression, thereby utilising fine details from the original impression where appropriate, and the fitted TASM model for infilling, extrapolation, correction of missing parts, and so on.

Figure 8 illustrates an example of annotating the TASM with meta data, in this case feature lines 80. Once the template is so annotated, this information is retained when the TASM is projected onto a new impression, eliminating the necessity to identify anew the features.

Other meta data that can be incorporated into the TASM include but are not limited to: meta data corresponding to anatomical features of the ear, such as shape features from the set of ear canal bends, aperture of ear canal, concha, cymba, helix, crus, inter-tragal notch, anterior notch, tragus, anti-tragus, ear drum, strong curvature lines, retention areas; contour lines; meta data corresponding to underlying bony structures, such as the transition between cartilaginous and bony ear canal parts or the jawbone influence area; meta data corresponding to intended positions of hearing aid components such as faceplates, receiver, microphone or microphones, hybrids, batteries, telephone coils, inductive coils, antenna coils, reed switches, capacitors, vents, and wax guards; or meta data corresponding to a pre-modelled deformable shape of at least part of a hearing device.

This latter is particularly useful in the manufacture of a custom hearing device using the above-mentioned method, since if the shape of the part of the hearing device, e.g. the shell of a hearing device intended to be inserted into the ear canal or an ear piece of a BTE device, is incorporated already as meta data into the TASM model, the shape of the hearing device can be automatically determined by warping fitting the TASM to the new impression, without further technician intervention.

It is self-evident that the above-mentioned methods are particularly suitable for determining an estimated shape of an individual ear for the purpose of manufacturing a custom hearing device. Using the method in this way results in a highly automated, accurate way of determining the shape of at least the portions of the hearing device destined coming contact with part of the wearer's ear, and it even produces good results based on fragmentary, incomplete, or otherwise low-quality measurement data.

In addition, the TASM-generating portion of the method is particularly usable on its own for generating a "best fit" ITE shell, BTE or CRT housing, or BTE sound tube that will fit a majority of people within an identifiable population group. The identifiable population group can be as described above, i.e. that of all persons, or defined by any combination of ethnicity, age ranges, sex, and so on. This template shape can then be used to define a part of a BTE/CRT housing that, when worn, will be in contact with the rear of the pinna of the wearer, or in the case of an ITE shell, that will be in contact with part of the ear of the wearer, and the housing can then be manufactured by injection moulding, or even CNC machining, 3-D printing or similar following the template shape where appropriate.

## Claims

1. Method of estimating the shape of an individual ear comprising the steps of:
a) determining part of the shape of the individual ear, the shape being determined over a first extent and constituting a determined shape (402, 502);
b) taking a predefined template shape (401, 501) determined by statistical analysis of a plurality of previously measured ear shapes according to a statistical model, the template shape (401, 501) having a second extent greater than the first extent;
c) generating an estimated shape (403, 503) corresponding to an estimated representation of the shape of the individual ear over a combination of the first extent and the second extent by modifying the,template shape (401, 501) to substantially match the determined shape (402, 502) over the intersection of the first extent and the second extent within a predefined tolerance;
wherein the previously measured ear shapes are obtained by at least one of CT scanning, MRI scanning, direct ear scanning, direct in-ear scanning, or a volumetric imaging technique, and
wherein the previously measured ear shapes comprise measurements up to the eardrum or to within 5 mm from the eardrum, and
wherein generating the estimated shape involves an estimation of the shape of the ear canal of the ear,
wherein the estimation of the- shape of the ear canal to within less than 5 mm of the eardrum or up to the eardrum is used to estimate at least one of the residual volume, or the Real Ear to Coupler Difference, or the Open Ear Gain.

2. Method according to claim 1, wherein the estimated shape (403, 503) is re-projected over the first extent onto the determined shape (402, 502) such that data from the estimated shape (403, 503) is used to interpolate, or extrapolate, or both, to thereby provide fill-in data for inhomogeneities caused by holes (94), fragmentary data, outlying points (92, 93, 95, 96), or missing parts of the determined shape (402, 502), wherein detail from the determined shape (402, 502) not captured in the estimated shape (403, 503) is incorporated into an infilled shape of the individual ear.

3. Method according to claim 2, wherein the extrapolation is used to estimate the shape of the ear canal up to the eardrum or to within less than 5 mm from the eardrum.

4. Method according to any preceding claim, wherein the template shape (401, 501) is provided with meta data corresponding to features, such as identifiable geometric features, texture, softness of tissues.

5. Method according to claim 4, wherein the meta data corresponds to anatomical features of the ear, namely one or more of: shape features from the set of ear canal bends, aperture of ear canal, concha, cymba, helix, crus, inter-tragal notch, anterior notch, tragus, anti-tragus, ear drum, strong curvature lines, and retention areas,
or wherein the meta data corresponds to contour lines,
or wherein the meta data corresponds to underlying bony structures, such as the transition between cartilaginous and bony ear canal parts or the jawbone influence area,
or wherein the meta data correspond to intended positions of hearing aid components, such as faceplates, receivers, microphone or microphones, hybrids, batteries, telephone coils, inductive coils, antenna coils, reed switches, capacitors, program switches, volume controls, vents, and wax guards.

6. Method according to claim 4 or 5, wherein the meta data corresponds to a pre-modelled deformable shape of at least part of a hearing device.

7. Method according to claim 4 or 5, wherein the meta data is transferred onto the estimated shape (403, 503), or when in combination with claim 2 onto the infilled shape, or both, as appropriate.

8. Method according to any preceding claim, wherein the template shape (401, 501) is selected from a plurality of previously determined template shapes corresponding to identifiable population groups, such as male, female, age ranges, ethnicity, or any combination thereof.

9. Method according to any preceding claim, wherein the template shape (401, 501) corresponds to at least part of the ear canal, at least part of the outer ear including the portion situated to the rear of the pinna, or both.

10. Method according to any preceding claim, wherein the statistical model is built by multidimensional Principle Component Analysis, also known as PCA, Covariance Analysis, Karhunen-Loeve Transform, or Hotelling Transform.

11. Method according to claim 10, wherein the determined shape (602) is incorporated if desired into the generation of the template shape (601) for the next time the method is carried out.

12. Method of manufacturing a custom fitted hearing device comprising the steps of:
- estimating the shape (403, 503) of an individual ear according to any preceding claim;
- manufacturing a shell of the custom fitted hearing device such that at least the portions of the shell destined to be in contact with part of the ear of the wearer are conformed according to the estimated or infilled shape,
wherein the shell manufactured by the method is a shell or earpiece shell of an ITE, CRT also known as RIC or RITE, BTE or HPD device, a swim plug, or a custom earphone.

13. Method of manufacturing a BTE or CRT housing, or a BTE tube to the shape and space available behind the ear of persons within an identifiable population group, comprising the steps of:
a) taking a predefined template (401, 501) shape determined by statistical analysis of a plurality of previously measured ear shapes according to a statistical model, the template shape (401, 501) corresponding to at least the shape and space behind the ear, the predefined template shape (401, 501) being obtained as in any one of claims 1 to 11;
b) defining a shape of at least part of the shape of the BTE or CRT housing or BTE tube corresponding to the predefined template shape (401, 501);
c) fabricating the housing according to the shape defined in step b).

14. Method of manufacturing an ITE shell to the shape of the ear of persons within an identifiable population group, comprising the steps of:
a) taking a predefined template shape (401, 501) determined by statistical analysis of a plurality of previously measured ear shapes according to a statistical model, the template shape (401, 501) corresponding to at least the part of the ear in which the ITE shell is destined to be situated in use, the predefined template shape (401, 501) being obtained as in any one of claims 1 to 11;
b) defining a shape of at least part of the shape of the ITE shell corresponding to the predefined template shape (401, 501);
c) fabricating the ITE shell according to the shape defined in step b).

15. Method according to claim 13 or 14, wherein the identifiable population group is at least on of male, female, age ranges, or ethnicity.

## Patentansprüche

1. Verfahren zum Schätzen der Form eines individuellen Ohrs, umfassend die folgenden Schritte:
a) Bestimmen eines Teils der Form des individuellen Ohrs, wobei die Form über eine erste Ausdehnung bestimmt wird und eine bestimmte Form (402, 502) bildet;
b) Abnehmen einer vordefinierten Schablonenform (401, 501), die durch statistische Analyse einer Vielzahl von zuvor gemessenen Ohrformen gemäß einem statistischen Modell bestimmt wird, wobei die Schablonenform (401, 501) eine zweite Ausdehnung aufweist, die größer als die erste Ausdehnung ist;
c) Erzeugen einer geschätzten Form (403, 503), die einer geschätzten Darstellung der Form des individuellen Ohrs über eine Kombination der ersten Ausdehnung und der zweiten Ausdehnung entspricht, indem die Schablonenform (401, 501) derart modifiziert wird, dass sie im Wesentlichen mit der bestimmten Form (402, 502) über die Schnittfläche der ersten Ausdehnung und der zweiten Ausdehnung innerhalb einer vordefinierten Toleranz übereinstimmt;
wobei die zuvor gemessenen Ohrformen durch wenigstens eines aus CT-Scannen, MRT-Scannen, direktes Scannen des Ohrs, direktes Scannen im Ohr oder eine Technik der volumetrischen Bildgebung gewonnen werden, und
wobei die zuvor gemessenen Ohrformen Messungen bis zum Trommelfell oder bis 5 mm vom Trommelfell umfassen, und
wobei das Erzeugen der geschätzten Form eine Schätzung der Form des Gehörgangs des Ohrs beinhaltet,
wobei die Schätzung der Form des Gehörgangs bis weniger als 5 mm vom Trommelfell oder bis zum Trommelfell verwendet wird, um wenigstens eines aus dem Restvolumen oder dem Real-Ear-to-Coupler-Difference-Wert oder dem Open-Ear-Gain-Wert zu schätzen.

2. Verfahren nach Anspruch 1, wobei die geschätzte Form (403, 503) über die erste Ausdehnung auf die bestimmte Form (402, 502) reprojiziert wird, so dass die Daten der geschätzten Form (403, 503) zur Interpolation oder Extrapolation oder zu beidem verwendet werden, um dadurch Ausfülldaten für Inhomogenitäten bereitzustellen, die durch Löcher (94), lückenhafte Daten, außerhalb gelegene Punkte (92, 93, 95, 96) oder fehlende Teile der bestimmten Form (402, 502) verursacht werden, wobei Einzelheiten der bestimmten Form (402, 502), die in der geschätzten Form (403, 503) nicht erfasst wurden, in eine ausgefüllte Form des individuellen Ohrs integriert werden.

3. Verfahren nach Anspruch 2, wobei die Extrapolation dazu verwendet wird, die Form des Gehörgangs bis zum Trommelfell oder bis weniger als 5 mm vom Trommelfell zu schätzen.

4. Verfahren nach einem vorhergehenden Anspruch, wobei die Schablonenform (401, 501) mit Metadaten versehen wird, die Merkmalen wie z.B. identifizierbaren geometrischen Merkmalen, Struktur, Weichheit von Geweben entsprechen.

5. Verfahren nach Anspruch 4, wobei die Metadaten anatomischen Merkmalen des Ohrs entsprechen, nämlich einem oder mehreren aus: Formmerkmalen des Satzes aus Gehörgangsbiegungen, Gehörgangsöffnung, Concha, Zymba, Helix, Crus, intertragaler Kerbe, vorderer Kerbe, Tragus, Antitragus, Trommelfell, starken Krümmungslinien und Rückhaltebereichen,
oder wobei die Metadaten Konturlinien entsprechen,
oder wobei die Metadaten darunterliegenden knochigen Strukturen wie z.B. dem Übergang zwischen knorpeligen und knochigen Gehörgangsteilen oder dem Kieferknocheneinflussbereich entsprechen,
oder wobei die Metadaten beabsichtigten Positionen von Hörgerätekomponenten wie z.B. Abdeckungen, Empfängern, dem Mikrofon oder Mikrofonen, Hybriden, Batterien, Telefonspulen, Induktionsspulen, Antennenspulen, Zungenschaltern, Kondensatoren, Programmschaltern, Lautstärkereglern, Lüftungen und Ohrenschmalzsperren entsprechen.

6. Verfahren nach Anspruch 4 oder 5, wobei die Metadaten einer vormodellierten verformbaren Form wenigstens eines Teils eines Hörgeräts entsprechen.

7. Verfahren nach Anspruch 4 oder 5, wobei die Metadaten auf die geschätzte Form (403, 503) oder, wenn in Kombination mit Anspruch 2, auf die ausgefüllte Form oder, wenn dies angemessen ist, auf beides übertragen werden.

8. Verfahren nach einem vorhergehenden Anspruch, wobei die Schablonenform (401, 402) aus einer Vielzahl von zuvor bestimmten Schablonenformen ausgewählt wird, die identifizierbaren Bevölkerungsgruppen wie z.B. Männern, Frauen, Altersbereichen, ethnischen Gruppen oder jeglichen Kombinationen davon entsprechen.

9. Verfahren nach einem vorhergehenden Anspruch, wobei die Schablonenform (401, 402) wenigstens einem Teil des Gehörgangs, wenigstens einem Teil des Außenohrs einschließlich des im hinteren Teil der Ohrmuschel gelegenen Abschnitts oder beidem entspricht.

10. Verfahren nach einem vorhergehenden Anspruch, wobei das statistische Modell durch mehrdimensionale Hauptkomponentenanalyse, auch bekannt als PCA, Kovarianzanalyse, Karhunen-Loeve-Transformation oder Hotelling-Transformation erstellt wird.

11. Verfahren nach Anspruch 10, wobei die bestimmte Form (602) wenn gewünscht in die Erzeugung der Schablonenform (601) integriert wird, wenn das Verfahren das nächste Mal ausgeführt wird.

12. Verfahren zur Herstellung eines individuell angepassten Hörgeräts, umfassend die folgenden Schritte:
- Schätzen der Form (403, 503) eines individuellen Ohrs gemäß einem vorhergehenden Anspruch;
- Herstellen einer Hülle des individuell angepassten Hörgeräts, so dass wenigstens die Abschnitte der Hülle, die dazu bestimmt sind, in Kontakt mit einem Teil des Ohrs des Trägers zu sein, gemäß der geschätzten oder ausgefüllten Form gestaltet sind,
wobei die durch das Verfahren hergestellte Hülle eine Hülle oder eine Ohrstück-Hülle für ein ITE-Gerät, ein auch als RIC- oder RITE-Gerät bekanntes CRT-Gerät, ein BTE-Gerät oder eine Gehörschutzvorrichtung, einen Schwimm-Ohrstöpsel oder einen individuell angepassten Kopfhörer ist.

13. Verfahren zur Herstellung eines BTE- oder CRT-Gehäuses oder eines BTE-Rohrs gemäß der Form und dem verfügbaren Raum hinter dem Ohr von Personen in einer identifizierbaren Bevölkerungsgruppe, umfassend die folgenden Schritte:
a) Abnehmen einer vordefinierten Schablonenform (401, 501), die durch statistische Analyse einer Vielzahl von zuvor gemessenen Ohrformen gemäß einem statistischen Modell bestimmt wird, wobei die Schablonenform (401, 501) wenigstens der Form und dem Raum hinter dem Ohr entspricht, wobei die vordefinierte Schablonenform wie in einem der Ansprüche 1 bis 11 gewonnen wird;
b) Definieren einer Form von wenigstens einem Teil der Form des BTE- oder CRT-Gehäuses oder des BTE-Rohrs entsprechend der vordefinierten Schablonenform (401, 501);
c) Herstellen des Gehäuses gemäß der in Schritt b) definierten Form.

14. Verfahren zum Herstellen einer ITE-Hülle gemäß der Form des Ohrs von Personen innerhalb einer identifizierbaren Bevölkerungsgruppe, umfassend die folgenden Schritte:
a) Abnehmen einer vordefinierten Schablonenform (401, 501), die durch statistische Analyse einer Vielzahl von zuvor gemessenen Ohrformen gemäß einem statistischen Modell bestimmt wird, wobei die Schablonenform (401, 501) wenigstens dem Teil des Ohrs entspricht, in dem die ITE-Hülle während des Gebrauchs angeordnet werden soll, wobei die vordefinierte Schablonenform (401, 501) wie in einem der Ansprüche 1 bis 11 gewonnen wird;
b) Definieren einer Form von wenigstens einem Teil der Form der ITE-Hülle, die der vordefinierten Schablonenform (401, 501) entspricht;
c) Herstellen der ITE-Hülle gemäß der in Schritt b) definierten Form.

15. Verfahren nach Anspruch 13 oder 14, wobei es sich bei der identifizierbaren Bevölkerungsgruppe um wenigstens eines aus Männern, Frauen, Altersgruppen oder ethnischen Gruppen handelt.

## Revendications

1. Procédé pour estimer la forme d'une oreille individuelle comprenant les étapes consistant à :
a) déterminer une partie de la forme de l'oreille individuelle, la forme étant déterminée sur une première étendue et constituant une forme déterminée (402, 502) ;
b) prendre une forme de modèle prédéfinie (401, 501) déterminée par une analyse statistique d'une pluralité de formes d'oreille mesurées précédemment selon un modèle statistique, la forme de modèle (401, 501) présentant une deuxième étendue plus grande que la première étendue ;
c) générer une forme estimée (403, 503) correspondant à une représentation estimée de la forme de l'oreille individuelle par une combinaison de la première étendue et de la deuxième étendue en modifiant la forme de modèle (401, 501) pour qu'elle corresponde sensiblement à la forme déterminée (402, 502) sur l'intersection de la première étendue et de la deuxième étendue dans une tolérance prédéfinie ;
dans lequel les formes d'oreilles mesurées précédemment sont obtenues par au moins un des procédés suivants : balayage CT, balayage IRM, balayage direct de l'oreille, balayage direct intra-auriculaire ou par une technique d'imagerie volumétrique, et
dans lequel les formes d'oreille précédemment mesurées comprennent des mesures jusqu'au tympan ou jusqu'à 5 mm du tympan, et
dans lequel la génération de la forme estimée consiste à estimer la forme du conduit auditif,
dans lequel l'estimation de la forme du conduit auditif à moins de 5 mm du tympan ou au niveau du tympan est utilisée pour estimer au moins le volume résiduel ou la différence entre l'oreille réelle et le coupleur ou le gain naturel de l'oreille.

2. Procédé selon la revendication 1, dans lequel la forme estimée (403, 503) est reprojetée sur la première étendue sur la forme déterminée (402, 502), de sorte que les données issues de la forme estimée (403, 503) sont utilisées pour interpoler, ou extrapoler, ou les deux, pour fournir des données de remplissage pour les inhomogénéités dues aux trous (94), données fragmentaires, points périphériques (92, 93, 95, 96) ou parties manquantes de la forme déterminée (402, 502),
dans lequel les détails issus de la forme déterminée (402, 502) non saisis dans la forme estimée (403, 503) sont intégrés dans une forme de remplissage de l'oreille individuelle.

3. Procédé selon la revendication 2, dans lequel l'extrapolation est utilisée pour estimer la forme du conduit auditif jusqu'au tympan ou jusqu'à moins 5 mm du tympan.

4. Procédé selon l'une des revendications précédentes, dans lequel la forme de modèle (401, 501) est fournie avec des métadonnées correspondant à des caractéristiques, telles que des caractéristiques géométriques identifiables, la texture, la souplesse des tissus.

5. Procédé selon la revendication 4, dans lequel les métadonnées correspondent à des caractéristiques anatomiques de l'oreille, notamment l'un des éléments suivants : caractéristiques de forme à partir des courbures du conduit auditif, orifice du conduit auditif, conque, cymba, hélix, crus, échancrure intertragienne, échancrure antérieure, tragus, antitragus, tympan, lignes de forte courbure et zones de rétention,
ou dans lequel les métadonnées correspondent aux lignes de contour,
ou dans lequel les métadonnées correspondent à des structures osseuses sous-jacentes, telles que la transition entre les parties cartilagineuses et les parties osseuses du conduit auditif ou la zone d'influence de la mâchoire,
ou dans lequel les métadonnées correspondent aux emplacements souhaités de composants d'appareils auditifs, tels que les façades, les récepteurs, le ou les microphones, les hybrides, les batteries, les bobines de téléphone, les bobines inductives, les bobines d'antennes, les commutateurs reed, les condensateurs, les commutateurs de programme, les commandes de volume, les fentes et les dispositifs anti-cérumen.

6. Procédé selon la revendication 4 ou 5, dans lequel les métadonnées correspondent à une forme déformable pré-modelée d'au moins une partie d'un dispositif auditif.

7. Procédé selon la revendication 4 ou 5, dans lequel les métadonnées sont transférées sur une forme estimée (403, 503) ou, lorsqu'il est en combinaison avec la revendication 2, sur la forme de remplissage, ou bien les deux selon le cas.

8. Procédé selon l'une des revendications précédentes, dans lequel la forme de modèle (401, 501) est sélectionnée parmi une pluralité de formes de modèle précédemment déterminées correspondant à des groupes de population identifiables, tels que les hommes, les femmes, les tranches d'âges, l'origine ethnique ou une combinaison de ces derniers.

9. Procédé selon l'une des revendications précédentes, dans lequel la forme de modèle (401, 501) correspond à au moins une partie du conduit auditif, au moins une partie de l'oreille externe, y compris la partie située à l'arrière du pavillon, ou bien les deux.

10. Procédé selon l'une des revendications précédentes, dans lequel le modèle statistique est élaboré par une analyse en composantes principales multidimensionnelle, connue également sous le nom de PCA, analyse de covariance, transformée de Karhunen-Loeve ou transformée de Hotelling.

11. Procédé selon la revendication 10, dans lequel la forme déterminée (602) est intégrée si on le souhaite dans la génération de la forme de modèle (601) pour la prochaine fois où l'on a recours au procédé.

12. Procédé pour fabriquer un appareil auditif sur mesure, comprenant les étapes consistant à :
- estimer la forme (403, 503) d'une oreille individuelle selon l'une des revendications précédentes,
- fabriquer une coque de l'appareil auditif sur mesure de sorte qu'au moins les parties de la coque destinées à être en contact avec une partie de l'oreille de l'utilisateur soient conformes à la forme estimée ou de remplissage,
dans lequel la coque fabriquée par le procédé est une coque ou une coque d'oreillette d'un dispositif ITE, CRT également connu sous le nom de dispositif RIC ou RITE, BTE ou HPD, un bouchon d'oreille de natation ou un écouteur individualisé.

13. Procédé pour fabriquer un boîtier BTE ou CRT, ou un tube BTE, à la forme et l'espace disponible derrière l'oreille de personnes dans un groupe de population identifiable, comprenant les étapes consistant à :
a) prendre une forme de modèle prédéfinie (401, 501) déterminée par une analyse statistique d'une pluralité de formes d'oreille précédemment mesurées selon un modèle statistique, la forme de modèle (401, 501) correspondant à au moins la forme et l'espace derrière l'oreille, la forme de modèle prédéfinie (401, 501) étant obtenue selon l'une des revendications 1 à 11 ;
b) définir une forme d'au moins une partie de la forme du boîtier BTE ou CRT ou du tube BTE correspondant à la forme de modèle prédéfinie (401, 501);
c) fabriquer le boîtier conformément à la forme définie à l'étape b).

14. Procédé pour fabriquer une coque ITE à la forme de l'oreille de personnes dans un groupe de population identifiable, comprenant les étapes consistant à :
a) prendre une forme de modèle prédéfinie (401, 501) déterminée par une analyse statistique d'une pluralité de formes d'oreille précédemment mesurées selon un modèle statistique, la forme de modèle (401, 501) correspondant à au moins une partie de l'oreille dans laquelle la coque ITE est destinée à être utilisée, la forme de modèle prédéfinie (401, 501) étant obtenue selon l'une des revendications 1 à 11 ;
b) définir une forme d'au moins une partie de la forme de la coque ITE correspondant à la forme de modèle prédéfinie (401, 501) ;
c) fabriquer la coque ITE conformément à la forme définie à l'étape b).

15. Procédé selon la revendication 13 ou 14, dans lequel le groupe de population identifiable est au moins un groupe d'hommes, de femmes, de tranches d'âge ou d'origine ethnique.
